# EUROPEAN PATENT SPECIFICATION

(11) **EP 3 167 882 B1**
(45) Date of publication and mention of the grant of the patent: **08.04.2020**
(21) Application number: 16185410.4
(22) Date of filing: 10.11.2006
(51) Int. Cl.: A61K 31/215, A61K 31/216, A61K 31/19, A61K 31/192, A61P 27/00

(54) **USE OF FENOFIBRATE OR A DERIVATIVE THEREOF FOR PREVENTING DIABETIC RETINOPATHY**
VERWENDUNG VON FENOFIBRAT ODER EINEM DERIVAT DAVON ZUR PRÄVENTION VON DIABETISCHER RETINOPATHIE
UTILISATION DU FÉNOFIBRATE OU L'UN DE SES DÉRIVÉS POUR LA PRÉVENTION DE LA RÉTINOPATHIE DIABÉTIQUE

(30) Priority: 10.11.2005 EP 05292388
(43) Date of publication of application: 17.05.2017
(62) Divisional of application: 06829976.7
(73) Proprietor: BGP Products Operations GmbH, 4123 Allschwil (CH)
(72) Inventor: ANSQUER, Jean-Claude, F-21000 Dijon (FR); KEECH, Anthony, Sydney, New South Wales (AU)
(74) Representative: FRKelly

(56) References cited:
- EP-A- 0 482 498
- EP-A- 1 325 745
- EP-A1- 1 714 648
- WO-A-02/080936
- WO-A-2004/052362
- WO-A-2004/066963
- WO-A-2005/074909
- US-A1- 2005 148 594
- FIELD STUDY INVESTIGATORS: "The need for a large-scale trial of fibrate therapy in diabetes: the rationale and design of the Fenofibrate Intervention and Event Lowering in Diabetes (FIELD) study. [ISRCTN64783481].", CARDIOVASCULAR DIABETOLOGY [ELECTRONIC RESOURCE]. 1 DEC 2004, vol. 3, 1 December 2004 (2004-12-01), page 9, XP002377468, ISSN: 1475-2840
- SCOTT R ET AL: "Fenofibrate Intervention and Event Lowering in Diabetes (FIELD) study: baseline characteristics and short-term effects of fenofibrate [ISRCTN64783481].", CARDIOVASCULAR DIABETOLOGY [ELECTRONIC RESOURCE]. 2005, vol. 4, 2005, page 13, XP002377469, ISSN: 1475-2840
- TENENBAUM ALEXANDER ET AL: "Which is the best lipid-modifying strategy in metabolic syndrome and diabetes: fibrates, statins or both?", CARDIOVASCULAR DIABETOLOGY [ELECTRONIC RESOURCE]. 1 DEC 2004, vol. 3, 1 December 2004 (2004-12-01), page 10, XP021007142, ISSN: 1475-2840

## Description

This invention relates to the use of fenofibrate or a derivative thereof for the manufacture of a medicament for the prevention and/or treatment of retinopathy.

Diabetes is a disorder in which the body is unable to metabolize carbohydrates (e.g., food starches, sugars, cellulose) properly. The disease is characterized by excessive amounts of sugar in the blood (hyperglycemia) and urine, inadequate production and/or utilization of insulin, and by thirst, hunger, and loss of weight. Diabetes affects about 2% of the population. Of these 10-15% are insulin dependant (type 1) diabetics and the remainder are non-insulin dependant (type 2) diabetics.

Diabetic retinopathy represents one of the most debilitating microvascular complications of diabetes. Diabetic retinopathy is a specific microvascular complication of both type 1 and type 2 diabetes. After 20 years of diabetes nearly all patients with type 1 diabetes and over 60% of patients with type 2 diabetes have some degree of retinopathy. Additionally, retinopathy develops earlier and is more severe in diabetics with elevated systolic blood pressure levels. On average, a careful eye examination reveals mild retinal abnormalities about seven years after the onset of diabetes, but the damage that threatens vision usually does not occur until much later. It can lead to blindness in its final stage. It is the second leading cause of acquired blindness in developed countries, after macular degeneration of the aged. The risk of a diabetic patient becoming blind is estimated to be 25 times greater than that of the general population. At present there is no preventive or curative pharmacological treatment for this complication. The only treatment is laser retinal photocoagulation or vitrectomy in the most severe cases.

Diabetic retinopathy is a progressive diabetic complication. It advances from a stage referred to as "simple" or initial (background retinopathy) to a final stage referred to as "proliferative retinopathy" in which there is formation of fragile retinal neovessels, leading to severe hemorrhages, sometimes with detachment of the retina, and to loss of vision. The microvascular lesions in simple retinopathy are characterized by microaneurysms, small petechial hemorrhages, exudates and venous dilations. This simple retinopathy form can remain clinically silent for a long period of time. At this simple retinopathy stage cellular and structural deterioration of the retinal capillary can be observed in the postmortem examinations of retinas from diabetic patients, compared to the retinas from normal subjects of comparable age. If proliferative retinopathy is left untreated, about half of those who have it will become blind within five years, compared to just 5% of those who receive treatment.

Diabetic retinopathy can be treated with laser photocoagulation, if it is detected early.

Laser treatment is usually carried out in a darkened room in a clinic; anaesthetic drops being dropped into the eye of the patient, a contact lens is placed on the eye of the patient, and then the patient is treated with a laser machine. Each treatment can be slightly different, depending on the condition of the eye. The laser can be pointed at one spot on the retina very accurately. Each bright flash can lasts 0.1 seconds or less. One of the commonest laser is Argon Green, wavelength 530 nm, but other wavelengths can be used which are equally effective.

For laser treatment of maculopathy an average of 100 burns may be needed, but this varies from very few to 250. More than one session may be needed.

For treatment of pre-proliferative retinopathy, severe pre-proliferative or proliferative retinopathy, each laser treatment is often 1000 burns or more. The side or 'peripheral' retina is lasered, not the centre; this is the main difference of laser for proliferative retinopathy as opposed to maculopathy. A 30 year old person with a lot of new vessels may need 6000 laser burns per eye, or even more, to prevent the new vessels growing.

Preventing the development or progression of diabetic retinopathy has the potential to save vision at a relatively low cost compared to the costs associated with a loss of vision. Thus, it is an object of the present invention to provide further means which contribute to the prevention of the development or progression of diabetic retinopathy.

The present invention is based on the discovery that patients taking fenofibrate or a derivative thereof need fewer treatment by retinal laser therapy than placebo-allocated patients. The results obtained from a large clinical trial demonstrate the favourable effect of fenofibrate in the prevention of retinopathy.

According to a first aspect, the present invention is directed to the use of fenofibrate or a derivative thereof for the manufacture of a medicament for the prevention and/or treatment of retinopathy, in particular diabetic retinopathy.

According to the present invention, "prevention" is defined as preventing the development or progression of diabetic retinopathy.

According to the present invention, "diabetic retinopathy" is defined as severe non proliferative grade of diabetic retinopathy, proliferative grades of diabetic retinopathy, macular edema and hard exsudates.

According to the present invention, fenofibrate or a derivative thereof can be used to prevent retinopathy. Fenofibrate is the 1-methylethyl ester (isopropyl ester) of fenofibric acid, i.e. fenofibrate (INN). As used herein, the term "fenofibric acid" refers to 2-[4-(4-chlorobenzoyl)phenoxy]-2-methyl-propanoic-acid.

A derivative of fenofibrate can be fenofibric acid, as well as a physiologically acceptable salt of fenofibric acid. The physiologically acceptable salts of the present invention are preferably base addition salts. The base addition salts include salts with inorganic bases, including, but not limited to, metal hydroxides or carbonates of alkali metals, alkaline earth metals or transition metals, or with organic bases, including, but not limited to, ammonia, basic amino acids such as arginine and lysine, amines, e.g. methylamine, dimethylamine, trimethylamine, triethyfamine, ethylamine, diethylamine, ethylenediamine, ethanolamine, diethanolamine, 1-amino-2-propanol, 3-amino-1-propanol or hexamethylenetetraamine, saturated cyclic amines having 4 to 6 ring carbon atoms, including, but not limited to, piperidine, piperazine, pyrrolidine and morpholine, and other organic bases, for example N-methylglucamine, creatine and tromethamine, and quaternary ammonium compounds, including, but not limited to, tetramethylammonium and the like. Salts with organic bases are preferably formed with amino acids, amines or saturated cyclic amines. Preferred salts with inorganic bases are preferably formed with Na, K, Mg and Ca cations.

In one embodiment of the invention, the salt of fenofibric acid is selected from the group consisting of choline, ethanolamine, diethanolamine, piperazine, calcium and tromethamine. These salts can be obtained according to the teaching of United States Patent Application 20050148594.

The medicament of the invention can be any kind of pharmaceutical formulation suitable for fenofibrate such as the formulations described in WO 00/72825 and the citations provided therein, such as U.S. Pat. Nos. 4,800,079, 4,895,726, 4,961,890, EP-A 0 793 958 and WO 82/01649. Additional formulations of fenofibrate are described in WO 02/067901 and citations provided therein, such as U.S. Pat. Nos. 6,074,670 and 6,042,847.

Besides the fenofibrate or derivative thereof, the formulations may comprise one or more other active substances, particularly those having an action like that of fenofibrate, e.g. selected from the group consisting of other lipid regulating agents, such as, but not limited to, further fibrates, e.g. bezafibrate, ciprofibrate and gemfibrocil, or statins, e.g. lovastatin, mevinolin, pravastatin, fluvastatin, atorvastatin, itavastatin, mevastatin, rosuvastatin, velostatin, synvinolin, simvastatin, cerivastatin and numerous others mentioned in, for instance, in WO 02/67901 and the corresponding citations therein as well as expedient active substances of other types.

The fenofibrate or derivative thereof ordinarily constitutes about 5 to about 60% by weight, preferably about 7 to about 40% by weight and, in particular, about 10 to about 30% by weight of the formulation. Data in % by weight are based, unless indicated otherwise, on the total weight of the formulation.

The formulations of the present invention comprise physiologically acceptable excipients. Physiologically acceptable excipients are those known to be usable in the pharmaceutical technology sectors and adjacent areas, particularly, those listed in relevant pharmacopeias (e.g. DAB, Ph. Eur., BP, NF, USP), as well as other excipients whose properties do not impair a physiological use.

Excipients are usually conventional pharmaceutical excipients, for example, fillers such as, but not limited to, sugar alcohols, e.g. lactose, microcrystalline cellulose, mannitol, sorbitol and xylitol, isomalt, starch saccharification products, talc, sucrose, cereal corn or potato starch, where present in a concentration of about 0.02 to about 50, preferably about 0.20 to about 20% by weight based on the total weight of the mixture; lubricants, glidants and mold release agents such as, but not limited to, magnesium, aluminum and calcium stearates, talc and silicones, and animal or vegetable fats, especially in hydrogenated form and those which are solid at room temperature; flow regulators, e.g. colloidal silica (highly dispersed silicon dioxide); dyes such as, but not limited to, azo dyes, organic or inorganic pigments or dyes of natural origin, with preference being given to inorganic pigments e.g. iron oxides, where present, in a concentration of about 0.001 to about 10, preferably about 0.1 to about 3% by weight, based on the total weight of the mixture; stabilizers such as, but not limited to, antioxidants, light stabilizers, hydroperoxide destroyers, radical scavengers, stabilizers against microbial attack; plasticizers, especially those described below. It is also possible to add wetting agents, preservatives, disintegrants, adsorbents and surfactants, especially anionic and nonionic, such as, for example, soaps and soap-like surfactants, alkyl sulfates and alkylsulfonates, salts of bile acids, alkoxylated fatty alcohols, alkoxylated alkylphenols, alkoxylated fatty acids and fatty acid glycerol esters, which may be alkoxylated, and solubilizers such as Cremophor® (polyethoxylated castor oil), Gelucire® and Labrafil® vitamin E TPGS and Tween® (ethoxylated sorbitan fatty acid esters). Excipients, for the purpose of the present invention, also refers to substances for producing a solid solution with the active substance. Examples of these excipients are pentaerythritol and pentaerythritol tetraacetate, urea, phosphatides such as lecithin, polymers such as, for example, polyethylene oxides and polypropylene oxides and their block copolymers (poloxamers) citric and succinic acids, bile acids, stearins and others as indicated, for example, by J. L. Ford, Pharm. Acta Hely. 61, (1986), pp. 69-88.

In one embodiment the pharmaceutical formulation usable according to the invention can contain an enteric binder which can be an enteric polymer, such as those selected from the group consisting of: hydroxypropylmethylcellulose phthalate, hydroxypropylmethyl-cellulose acetate succinate, carboxymethylethylcellulose, cellulose acetate phthalate, cellulose acetate trimellitate and carboxymethylcellulose sodium. Additionally, the enteric polymer can be a copolymer, such as a copolymer of (meth)acrylic acid and at least one alkyl (meth)acrylic acid ester. The alkyl (meth)acrylic acid ester can be methyl methacrylate. The copolymer can have a ratio of free carboxyl groups to esteritied carboxyl groups of about 2:1 to 1:3, preferably, about 1:1.

The pharmaceutical formulations of the present invention are mainly used in the physiological practice, particularly, in the medical sector for human. In this sense, the formulations are used as or in dosage forms, i.e. the formulations of the present invention have expedient forms that are appropriate for physiological practice, if necessary together with other excipients. Thus, the term "dosage form" refers to any dosage form that is suitable for administration of active substances to humans.

Conventional dosage forms include, but are not limited to capsules, granules, pellets, powders, suspensions, suppositories, tablets. Granules comprise solid grains of the formulations of the present invention, wherein each grain represents an agglomerate of powder particles. Granules can have a mean com size in the range of about 0.12 to about 2 mm, preferably about 0.2 to about 0.7 mm. Granules are preferably intended for oral use as dosage forms. The user can be offered single-dose preparations, for example, granules packed in a small bag (sachet), a paper bag or a small bottle, or multidose preparations which require appropriate measuring, such granules do not represent the actual dosage form, but are intermediates in the manufacture of particular dosage forms, for example, tablet granules to be compressed to tablets, capsule granules to be packed into hard gelatin capsules, or instant granules or granules for oral suspension to be put in water before intake.

As capsules, the formulations of the present invention are usually packed into a hard shell composed of two pieces fitted together or a soft, one-piece, closed shell, which may vary in shape and size. It is possible for the formulations of the present invention to be encased or enveloped or embedded in a matrix in suitable polymers, that is to say, microcapsules and microspherules. Hard and soft capsules comprise mainly of gelatin, while the latter can have a suitable content of plasticizing substances such as glycerol or sorbitol. Hard gelatin capsules are used to receive formulations of the present invention that have a solid consistency, for example granules, powder or pellets. Soft gelatin capsules are suitable for formulations with a semisolid consistency and, if required, also viscous liquid consistency.

In semisolid preparations, formulations of the present invention are taken up in a suitable vehicle. Appropriate bases are known to those skilled in the art.

Suppositories are solid preparations for rectal, vaginal or urethral administration. In order to be appropriate for this route of administration, formulations of the present invention in these drug forms must be taken up in suitable vehicles, for example, in fats which melt at body temperature, such as hard fat, macrogols, i.e. polyethylene glycols with molecular weights of about 1000 to about 3000 in various proportions, glycerol, gelatin and the like.

Tablets are solid preparations for oral use. The meaning of oral within the framework of the present invention is, particularly, that of the term "peroral", i.e. tablets for absorption or action of the active substance in the gastrointestinal tract. Particular embodiments include, but are not limited to, coated tablets, layered tablets, laminated tablets, tablets with modified release of active substance, matrix, tablets, effervescent tablets or chewable tablets. The formulations of the present invention usually comprise at least a part of the necessary tablet excipients, such as binders, fillers, glidants and lubricants, and disintegrants. Tablets of formulations of the present invention may also, if necessary, comprise other suitable excipients. Excipients which assist tableting, for example lubricants and glidants, for example those mentioned above, with preference for flow regulators such as silica and/or lubricants such as magnesium stearate, particularly for facilitating compaction, can also be used herein. Coated tablets additionally comprise suitable coating materials, for example, film coating agents with coating aids, especially those mentioned below. Coated tablets include, but are not limited to, sugar-coated tablets and film-coated tablets,

The formulations of the present invention, when used as a dosage form and thus providing an effective amount of active substance, are administered to the individual to be treated. Whether such a treatment is indicated and what form it is to take depends on the individual case and may be subject to medical assessment (diagnosis) which includes the signs, symptoms and/or dysfunctions which are present, the risks of developing certain signs, symptoms and/or dysfunctions, and other factors.

These formulations are ordinarily administered together or alternately with other products in such a way that an individual to be treated receives a daily dose of about 50 mg to about 250 mg fenofibrate on oral administration. In one embodiment of the invention the daily dose is 200 mg, 160 mg, 145 mg or 130 mg.

Any delivery route can be employed for providing a patient with an effective amount of fenofibrate or a derivative thereof. Preferred delivery routes include, but are not limited to oral or parenteral, e.g., rectal, vaginal, urethral, topical, the oral delivery route being preferred.

By way of example, and not of limitation, examples of the present invention will now be given.

### PHARMACOLOGICAL DATE

Type 2 diabetes mellitus is an increasingly common condition associated with a high cardiovascular risk. To date, very few trials of lipid-lowering therapy have focused on this condition, and in particular, no very large trials of fibrate therapy in diabetes have been conducted. Only two trials of fibrate therapy have been completed: the Veterans Low-HDL Cholesterol Intervention Trial (VA-HIT) Rubins HB, Robins SJ, Collins D, Fye CL, Anderson JW, Elam MB, Faas FH, Linares E, Schaefer EJ, Schectman G, Wilt TJ, Wittes J: Gemfibrozil for secondary prevention of coronary heart disease in men with low levels of high-density lipoprotein cholesterol. Veterans Affairs High-density liporotein cholesterol Intervention Trial study group. N. Engl J Med 1999, 341 : 410-418) and the Bezafibrate Infarct Prevention (BIP) trial (The RIP Study Group : Secondary Prevention by raising HDL cholesterol and reducing triglycerides in patients with coronary artery disease. The Bezafibrate Infarct Prevention (BIP) Study. Circulation 2000, 102:21-27). Both studies were limited to people with prior myocardial infarction (MI) and have reported reductions in major cardiovascular events among participants with low HDL and high triglyceride (TG) at baseline, which were greater than those seen with use of the same fibrate among those without dyslipidaemia. The VA-HIT trial also reported reduced coronary heart disease (CHD) mortality in those with diabetes receiving gemfibrozil and a reduced rate of cardiovascular events, although rates of nonfatal MI did not change significantly. A third trial, the Diabetes Atherosclerosis Intervention Study (DAIS), showed reduced progression of established coronary atherosclerosis among those randomized to fenofibrate compared with those receiving matching placebo, over 3 years. (Diabetes Atherosclerosis Intervention Study investigators : Effect of fenofibrate on progression of coronary artery disease in type 2 diabetes : the Diabetes Atherosclerosis intervention Study, a randomised study. Lancet 2001, 357:905-910).

As fibrates are known to correct the typical dyslipidaemia of diabetes, their role in cardiovascular risk reduction in diabetes may be especially important. A study called Fenofibrate Intervention and Event Lowering in Diabetes (FIELD) has been carried out which study is a multicentre, double-blind, placebo-controlled trail evaluating the effects on coronary morbidity and mortality of long-term treatment with fenofibrate to elevate high-density lipoprotein (HDL) cholesterol levels and lower triglyceride (TG) levels in patients with type 2 diabetes and total blood cholesterol between 3 and 6.5 mmol/L (115 and 250 mg/dL) at study entry. In type 2 diabetes, rates of coronary heart disease (CDH) are 3 to 4 times higher than those of persons without diabetes at any given level of blood cholesterol, and at any given age. Evidence also suggests that in women with diabetes, the natural protection against CDH afforded by sex may be lost. Further, people with type 2 diabetes have both higher in hospital mortality after myocardial infarction (MI) and a poorer outcome in the subsequent years, losing on average between 5 and 10 years of life expectancy. It follows that type 2 diabetes contributes significantly to the overall burden of premature CHD morbidity and mortality, far in excess of its prevalence in the community.

Blood total cholesterol levels are not substantially different between patients with type 2 diabetes and those of nondiabetic populations of similar age and sex. However, evaluation of other lipoprotein fractions shows that those with diabetes more often have a below-average HDL cholesterol level and elevation of TG levels in the blood which together confer an independent additionnal risk of CHD. Furthermore, although low-density lipoprotein (LDL) cholesterol levels are not substantially raised, the HDL particle is often smaller and denser than in similar nondiabetic populations, which is considered to be a more atherogenic state. An increased number of LDL particles, as seen in diabetes, is reflected in an elevated level of plasma apolipoprotein B, a more powerful predictor of risk for cardiovascular events than either total cholesterol or LDL cholesterol.

The strength of cholesterol-CHD relationship is very similar for those with type 2 diabetes as for nondiabetics, although at a higher background rate of CHD. Evidence from Helsinki Heart Study, which tested long-term fibrate (gemfibrozil) use in hypercholesterolaemic men and women without prior coronary disease, showed a significant reduction in coronary events, with the reduction among the small numbers of people with diabetes not being separately significant but appearing somewhat greater. The reductions in events observed were greater than would have been expected on the basis of lowering of LDL cholesterol alone. So, whether substantially increasing low HDL cholesterol levels and reducing elevated triglyceride levels independently reduces cardiovascular events and mortality and should be a specific target for therapy remains less well agreed.

For patients with type 2 diabetes and its typical dyslipidaemia, many physicians believe that fibrates are the logical first choice of drug treatment. The fibrates have been in clinical use for a long time, being well tolerated and with few short-term side -effects. Fenofibrate has been widely used and marketed for more than 20 years and is an effective agent for reducing plasma triglyceride and raising HDL cholesterol. Although the effects on lipid fractions may vary with the population under study, a fall of 15 % or more in total cholesterol, mediated through a reduction in LDL cholesterol, is often seen with long-term use. In parallel, HDL cholesterol elevation of 10-15 % is common, together with large reductions in plasma triglycerides of 30-40 %. In addition, a reduction in plasma fibrinogen of about 15 % has been observed.

The study was designed to provide the first properly randomized evidence as to whether the substantial effects of fenofibrate confer a benefit on clinical cardiovascular events in persons with type 2 diabetes.

The study was is randomized, double-blind, placebo-controlled parallel-group trial among middle-aged to elderly people with type 2 diabetes mellitus considered to be at increased risk of CHD. Those with and without pre-existing vascular disease or other lipid abnormalities, such as low HDL cholesterol and elevated TG, were eligible, provided the total blood cholesterol level at screening fell between 3.0 and 6.5 mmol/L (about 115-250 mg/dL) plus either a total-to-HDL cholesterol ratio of >4.0 or a blood TG level >1.0 mmol/L (88.6 mg/ dL). The study has been conducted in 63 clinical centres in Australia (39), Finland (9) and New Zealand (15).

The underlying principle guiding recruitment of patients into the study was that of clinical uncertainty : that is, patients were only to be considered if the patients' treating physicians were substantially uncertain about the value of lipid-modifying therapy for that particular individual and felt that there was no indication for lipid-modifying therapy. Therefore, none of the participants was on lipid-lowering therapy at study entry.

Following clinical and laboratory screening for eligibility, informed consent, and completion of the run-in period, patients were randomized to receive either fenofibrate (200 mg comicronized formulation) or matching placebo as one capsule daily breakfast. There was no formal restriction on randomization related to compliance during the run-in period. Randomization was carried out using a dynamic allocation method with stratification for important prognostic factors, including age, sex, prior MI, lipid levels and urinary albumin excretion. All patients were followed up through regular clinic visits to their usual specialist diabetes clinic or to a clinic set in place for the purposes of the study.

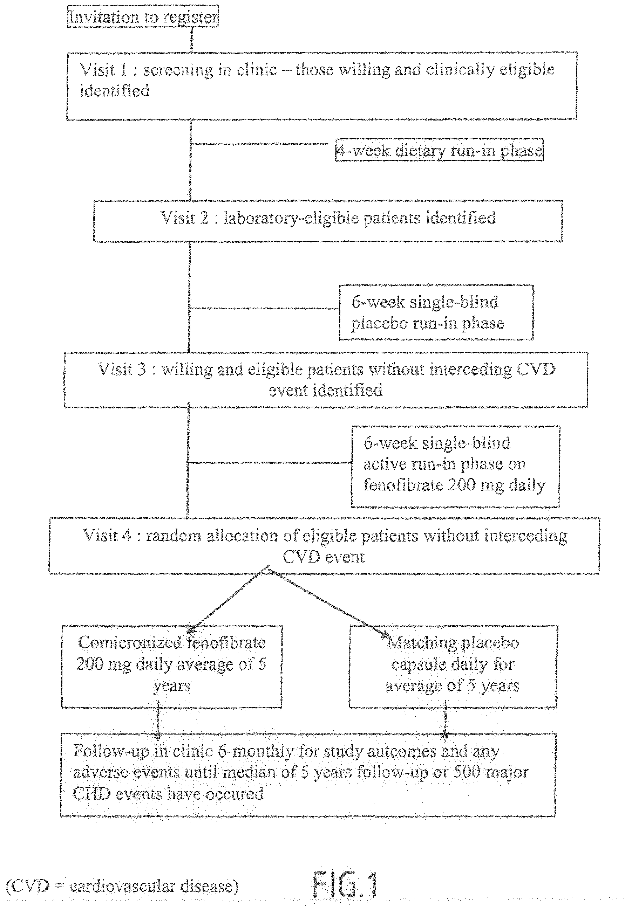

The run-in phase for the study consists of a 4-week diet only period, followed by a 6-week single-blind placebo period, then a 6-week single-blind active run-in period on comicronized fenofibrate 200 mg once daily for all patients, before randomization (figure 1). This was to allow patients time to discuss long-term participation with their families and their usual doctors and for evaluation of the benefits of fenofibrate treatment on a background of recommended dietary advice. Further, the active run-in period was to determine to what extent any long-term clinical benefits of treatment correlate with the short-term effects of the drug to modify different lipid fractions.

Follow-up in the study was more than 5 years.

The principal study outcome is the combined incidence of first nonfatal MI or CHD death among all randomized patients during the schedule treatment period. Secondary outcomes include the effects fenofibrate on major cardiovascular events (CHD events, total stroke and other cardiovascular death combined), total cardiovascular events (major cardiovascular events plus coronary and carotid revascularization), CHD death, total cardiovascular deaths, haemorrhagic and nonhaemorrhagic stroke, coronary and peripheral revascularization procedures, cause-specific non-CHD mortality (including cancer, suicide), and total mortality. All deaths, possible MIs and possible strokes are adjudicated in blinded fashion by the Outcomes Assessment Committee. Tertiary outcomes include the effects of treatment on development of vascular and neuropathic amputations, nonfatal cancers, the progression of renal disease, laser treatment for diabetic retinopathy, hospitalization for angina pectoris, and numbers and duration of all hospital admissions.

Sample size: Of the 13 900 patients screened in study clinics, 75.9 % (10 553) proceeded to enter the placebo run-in phase and 73.4 % (10 202) the active run-in phase, and ultimately 9 795 (70.5 %) patients were randomized. The demographic characteristics are shown for the randomized and nonrandomized patients in Table 1. The study cohort included more men than women, and had a mean age in the mid-60s, and just over 2100 randomized patients had a prior history of cardiovascular disease.

There were 6051 subjects randomized from Australia, 2 351 from New Zealand and 1 393 from Finland. There were no differences across the 3 countries in mean ages of patients or ages at diagnosis (55.8 years and 56.2 years for Australia, Finland and New Zealand, respectively). The median periods from diabetes diagnosis to randomization were 5, 7 and 5 years, respectively. The patients recruited were a mix from hospital and the community, and therefore were diverse in absolute risk.

**Table 1 : baseline characteristics of the FIELD study cohort**

| **Characteristic** | **Category** | **Screened, not randomized (n=4105)** | | **Randomized (n-9795)** | |
|---|---|---|---|---|---|
| | | n | % | n | % |
| Sex | Male | 2424 | 59.0 | 6138 | 62.7 |
| | Female | 1681 | 41.0 | 3657 | 37.3 |
| Age at visit 1 (years) | > 55 | 682 | 16.6 | 1668 | 17.0 |
| | 55-59 | 802 | 19.5 | 1976 | 20.2 |
| | 60-64 | 856 | 20.8 | 2196 | 22.4 |
| | 65-69 | 888 | 21.7 | 2202 | 22.5 |
| | 70+ | 877 | 21.4 | 1753 | 17.9 |
| Ethnicity* | Caucasian | 3757 | 91.5 | 9093 | 92.8 |
| | Indigenous | 161 | 3.9 | 258 | 2.6 |
| | Asian | 93 | 2.3 | 141 | 1.4 |
| | Other | 94 | 2.3 | 303 | 3.1 |
| Body mass index | BMI < 25 | 814 | 19.8 | 1242 | 12.7 |
| (kg/cm²) (BMI) | 25 ≤ BMI <30 | 1505 | 36.7 | 3805 | 38.9 |
| | 30 ≤ BMI < 35 | 1096 | 26.7 | 2885 | 29.5 |
| | BMI ≥ 35 | 652 | 15.9 | 1853 | 18.9 |
| Waist (cm) | Female (mean, SD) | 98±15 | | 101±14 | |
| | Male (mean, SD) | 103±13 | | 105±12 | |
| Waist-hip ratio | Ratio < 0.8 | 268 | 6.5 | 375 | 3.8 |
| | 0.8 ≤ ratio < 0.9 | 1248 | 30.4 | 2668 | 27.2 |
| | 0.9 ≤ ratio < 1.0 | 1840 | 44.8 | 4764 | 48.6 |
| | 1.0 ≤ ratio < 1.1 | 651 | 15.9 | 1809 | 18.5 |
| | Ratio ≥ 1.1 | 68 | 1.7 | 167 | 1.7 |
| Smoking (cigarettes) | Nonsmoker | 1735 | 42.3 | 4000 | 40.8 |
| | Ex-smoker | 1933 | 47.1 | 4908 | 50.1 |
| | Current smoker | 404 | 9.8 | 887 | 9.1 |
| Clinical history | Prior cariovascular disease † | 1044 | 25.4 | 21.31 | 21.8 |
| | Prior myocardial infarction | 278 | 6.7 | 484 | 5.0 |
| | Stroke | 193 | 4.7 | 346 | 3.5 |
| | Angina | 588 | 14.3 | 1188 | 12.1 |
| | Hypertension | 2246 | 54.7 | 5548 | 56.6 |
| | Claudication of peripheral vascular disease | 351 | 8.6 | 710 | 7.2 |
| | Transient ischemic attack | 155 | 3.8 | 307 | 3.1 |
| Prior coronary | No CABG or PTCA | 3908 | 95.2 | 9432 | 96.3 |
| revascularisation | CABG only | 122 | 3.0 | 230 | 2.4 |
| | PTCA only | 49 | 1.2 | 102 | 1.0 |
| | CABG and PTCA | 26 | 0.6 | 31 | 0.3 |
| Diabetes | Diet only | 1103 | 26.9 | 2675 | 27.3 |
| management | Diet + OH only | 2237 | 54.5 | 5828 | 59.5 |
| | Diet + insulin | 447 | 10.9 | 601 | 6.1 |
| | Diet + OH+insulin | 318 | 7.7 | 691 | 7.1 |
| Diabetic | Retinopathy | 430 | 10.5 | 813 | 8.3 |
| Complications ‡ | Neuropathy | 636 | 15.5 | 1395 | 14.2 |
| | Nephropathy | 160 | 3.9 | 279 | 2.9 |
| | Skin ulcers | 139 | 3.4 | 299 | 3.1 |
| | Amputations | 106 | 2.6 | 176 | 1.8 |
| Diabetes diagnosis ‡ | Age at diagnosis (mean ± SD) | 54.8±8.7 | | 55.5±8.3 | |
| | Median duration of diabetes in years (quartile1, quartile 3) | 6(2,11) | | 5(2,10) | |

| | | | | | |
|---|---|---|---|---|---|
| * patients are assumed to be Caucasian unless otherwise stated. Indigenous includes Aborigines, Torres Strait Islanders, Maori and Pacific Islanders. Other includes mixed races African-American , Indian, African, etc. † Prior cardiovascular disease is defined as a reported history of myocardial infarction, angina (stable and unstable), coronary revascularization (CABG or PTCA), stroke, claudication or peripheral vascular disease or peripheral revascularization before randomization. The count includes 13 randomized patients and 10 nonrandomized patients who suffered or reported a cardiovascular event during the run-in period (visit 1 to visit 4 (randomization)). ‡ Diabetic complications and age and diabetes diagnosis are self-reported. CABG = coronary artery bypass grafting; PTCA = percutaneous transluminal coronary angioplasty; SD = standard deviation; OH = oral hypoglycemic agents. | | | | | |

### Results for tertiary outcome related to laser treatment for diabetic retinopathy :

**1. Number of patients with laser treatment for diabetic retinopathy**

| | Placebo | | Fenofibrate | | Total | |
|---|---|---|---|---|---|---|
| | N | % | N | % | N | % |
| Total number of patients | 253 | 5.2 | 178 | 3.6 | 431 | 4.4 |

These results show that more located patients (253 (5.2%)) than fenofibrate-allocated patients (178 (3.6%)) required one or more laser treatments for retinopathy (p<0.001).

**2. Incidence and prevalence of laser treatment for diabetic retinopathy**

| | Placebo | | Fenofibrate | | Total | |
|---|---|---|---|---|---|---|
| | N | % | N | % | N | % |
| Incident cases | 191 | 3.9 | 115 | 2.3 | 306 | 4.4 |
| Prevalent cases | 556 | 11.3 | 355 | 7.3 | 911 | 9.3 |

The incident cases correspond to the first laser treatment for retinopathy post-randomisation.

The prevalent cases correspond to all reported laser treatments post-randomisation.

Thus, the effect of fenofibrate was very similar among just those patients without retinopathy at baseline (p=0.001).

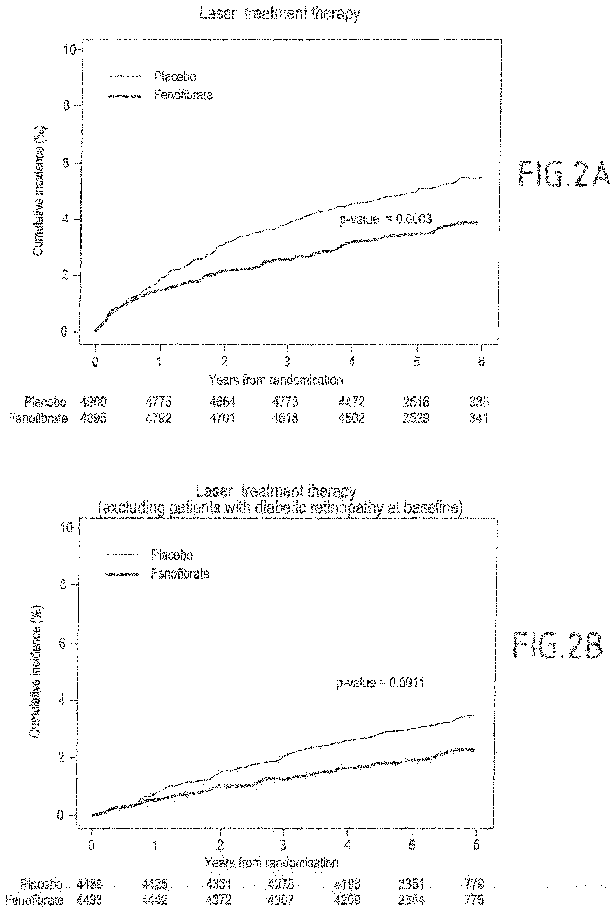

Figure 2A and 2B are Kaplan-Meier curves showing the cumulative incidence of laser treatment therapy with and without the patients with diabetic retinopathy at baseline

These results provide the first evidence of the favourable effect of fenofibrate on the need for retinal laser therapy. As the patients taking fenofibrate had fewer treatment by retinal laser therapy than the placebo-allocated patients, the prevention and treatment of retinopathy by fenofibrate has been clearly demonstrated.

## Claims

1. Use of fenofibrate, fenofibric acid or a physiologically acceptable salt of fenofibric acid for the manufacture of a medicament for the prevention of retinopathy, in particular diabetic retinopathy.

2. The use according to claim 1, wherein the physiologically acceptable salt of fenofibric acid is selected from the group consisting of choline, ethanolamine, diethanolamine, piperazine, calcium and tromethamine.

3. The use according to claim 1 or 2, wherein said medicament further contains a statin.

4. The use according to any of claims 1 to 3, wherein said medicament contains 200 mg, 160 mg, 145 mg or 130 mg of fenofibrate or fenofibric acid or a physiologically acceptable salt of fenofibric acid.

5. The use according to any of claims 1 to 4, wherein said medicament is an oral formulation of fenofibrate or fenofibric acid or a physiologically acceptable salt of fenofibric acid.

6. Fenofibrate, fenofibric acid or a physiologically acceptable salt of fenofibric acid for use in the prevention and/or treatment of retinopathy, in particular diabetic retinopathy.

7. Fenofibrate, fenofibric acid or a physiologically acceptable salt of fenofibric acid for use according to claim 6, wherein the physiologically acceptable salt of fenofibric acid is selected from the group consisting of choline, ethanolamine, diethanolamine, piperazine, calcium and tromethamine.

8. Fenofibrate, fenofibric acid or a physiologically acceptable salt of fenofibric acid for use according to claim 6 or 7, wherein said fenofibrate, fenofibric acid or a physiologically acceptable salt of fenofibric acid further comprises a statin.

9. Fenofibrate, fenofibric acid or a physiologically acceptable salt of fenofibric acid for use according to any of claims 6 to 8, wherein said fenofibrate, fenofibric acid or a physiologically acceptable salt of fenofibric acid comprises 200 mg, 160 mg, 145 mg or 130 mg of fenofibrate or fenofibric acid or a physiologically acceptable salt of fenofibric acid.

10. Fenofibrate, fenofibric acid or a physiologically acceptable salt of fenofibric acid for use according to any of claims 6 to 9, wherein said fenofibrate, fenofibric acid or a physiologically acceptable salt of fenofibric acid is an oral formulation of fenofibrate or fenofibric acid or a physiologically acceptable salt of fenofibric acid.

## Patentansprüche

1. Verwendung von Fenofibrat, Fenofibrinsäure oder einem physiologisch verträglichen Salz von Fenofibrinsäure zur Herstellung eines Medikaments zur Prävention von Retinopathie, insbesondere diabetischer Retinopathie.

2. Verwendung nach Anspruch 1, wobei das physiologisch verträgliche Salz von Fenofibrinsäure ausgewählt ist aus der Gruppe, bestehend aus Cholin, Ethanolamin, Diethanolamin, Piperazin, Calcium und Tromethamin.

3. Verwendung nach Anspruch 1 oder 2, wobei das Medikament ferner ein Statin enthält.

4. Verwendung nach einem der Ansprüche 1 bis 3, wobei das Medikament 200 mg, 160 mg, 145 mg oder 130 mg Fenofibrat oder Fenofibrinsäure oder ein physiologisch verträgliches Salz von Fenofibrinsäure enthält.

5. Verwendung nach einem der Ansprüche 1 bis 4, wobei es sich bei dem Medikament um eine orale Formulierung aus Fenofibrat oder Fenofibrinsäure oder einem physiologisch verträglichen Salz von Fenofibrinsäure handelt.

6. Fenofibrat, Fenofibrinsäure oder ein physiologisch verträgliches Salz von Fenofibrinsäure zur Verwendung bei der Prävention und/oder Behandlung von Retinopathie, insbesondere diabetischer Retinopathie.

7. Fenofibrat, Fenofibrinsäure oder ein physiologisch verträgliches Salz von Fenofibrinsäure zur Verwendung nach Anspruch 6, wobei das physiologisch verträgliche Salz von Fenofibrinsäure ausgewählt ist aus der Gruppe, bestehend aus Cholin, Ethanolamin, Diethanolamin, Piperazin, Calcium und Tromethamin.

8. Fenofibrat, Fenofibrinsäure oder ein physiologisch verträgliches Salz von Fenofibrinsäure zur Verwendung nach Anspruch 6 oder 7, wobei das Fenofibrat, die Fenofibrinsäure oder ein physiologisch verträgliches Salz von Fenofibrinsäure ferner ein Statin umfasst.

9. Fenofibrat, Fenofibrinsäure oder ein physiologisch verträgliches Salz von Fenofibrinsäure zur Verwendung nach einem der Ansprüche 6 bis 8, wobei das Fenofibrat, die Fenofibrinsäure oder ein physiologisch verträgliches Salz von Fenofibrinsäure 200 mg, 160 mg, 145 mg oder 130 mg Fenofibrat oder Fenofibrinsäure oder physiologisch verträgliches Salz von Fenofibrinsäure umfasst.

10. Fenofibrat, Fenofibrinsäure oder ein physiologisch verträgliches Salz von Fenofibrinsäure zur Verwendung nach einem der Ansprüche 6 bis 9, wobei es sich bei dem Fenofibrat, der Fenofibrinsäure oder einem physiologisch verträglichen Salz von Fenofibrinsäure um eine orale Formulierung aus Fenofibrat oder Fenofibrinsäure oder einem physiologisch verträglichen Salz von Fenofibrinsäure handelt.

## Revendications

1. Utilisation de fénofibrate, d'acide fénofibrique ou d'un sel physiologiquement acceptable de l'acide fénofibrique pour la fabrication d'un médicament destiné à la prévention de la rétinopathie, en particulier la rétinopathie diabétique.

2. Utilisation selon la revendication 1, dans laquelle le sel physiologiquement acceptable de l'acide fénofibrique est choisi parmi le groupe constitué de choline, d'éthanolamine, de diéthanolamine, de pipérazine, de calcium et de trométhamine.

3. Utilisation selon la revendication 1 ou 2, dans laquelle ledit médicament contient en outre une statine.

4. Utilisation selon l'une quelconque des revendications 1 à 3, dans laquelle ledit médicament contient 200 mg, 160 mg, 145 mg ou 130 mg de fénofibrate ou d'acide fénofibrique ou d'un sel physiologiquement acceptable de l'acide fénofibrique.

5. Utilisation selon l'une quelconque des revendications 1 à 4, dans laquelle ledit médicament est une formulation orale de fénofibrate ou d'acide fénofibrique ou d'un sel physiologiquement acceptable de l'acide fénofibrique.

6. Fénofibrate, acide fénofibrique ou sel physiologiquement acceptable de l'acide fénofibrique pour une utilisation dans la prévention et/ou le traitement de la rétinopathie, en particulier la rétinopathie diabétique.

7. Fénofibrate, acide fénofibrique ou sel physiologiquement acceptable de l'acide fénofibrique à utiliser selon la revendication 6, dans lequel le sel physiologiquement acceptable de l'acide fénofibrique est choisi dans le groupe constitué de choline, d'éthanolamine, de diéthanolamine, de pipérazine, de calcium et de trométhamine.

8. Fénofibrate, acide fénofibrique ou sel physiologiquement acceptable de l'acide fénofibrique pour une utilisation selon la revendication 6 ou 7, dans lequel le fénofibrate, l'acide fénofibrique ou un sel physiologiquement acceptable de l'acide fénofibrique comprend en outre une statine.

9. Fénofibrate, acide fénofibrique ou sel physiologiquement acceptable de l'acide fénofibrique pour une utilisation selon l'une quelconque des revendications 6 à 8, dans lequel le fénofibrate, l'acide fénofibrique ou un sel physiologiquement acceptable de l'acide fénofibrique comprend 200 mg, 160 mg, 145 mg ou 130 mg de fénofibrate ou d'acide fénofibrique ou d'un sel physiologiquement acceptable de l'acide fénofibrique.

10. Fénofibrate, acide fénofibrique ou sel physiologique acceptable de l'acide fénofibrique pour une utilisation selon l'une quelconque des revendications 6 à 9, dans lequel le fénofibrate, l'acide fénofibrique ou un sel physiologiquement acceptable de l'acide fénofibrique est une formulation orale de fénofibrate ou d'acide fénofibrique ou d'un sel physiologiquement acceptable de l'acide fénofibrique.
